# FASCICULE DE BREVET EUROPEEN

(11) **EP 0 533 588 B1**
(45) Date de publication et mention de la délivrance du brevet: **31.08.1994**
(21) Numéro de dépôt: 92402581.0
(22) Date de dépôt: 18.09.1992
(51) Int. Cl.: A61B 6/00, A61B 6/04

(54) **Dispositif de compression controlée du sein pour mammographe**
Gesteuerte Brustkompressionseinrichtung für Mammograph
Controlled breast compression means for a mammograph

(30) Priorité: 19.09.1991 FR 9111570
(43) Date de publication de la demande: 24.03.1993
(73) Titulaire: GE MEDICAL SYSTEMS S.A., F-92130 Issy les Moulineaux (FR)
(72) Inventeur: Saladin, Jean-Pierre, F-75116 Paris (FR); Pelissonnier, Bernard, F-75116 Paris (FR)
(74) Mandataire: Lemoyne, Didier

(56) Documents cités:
- EP-A- 0 144 670
- EP-A- 0 406 455
- EP-A- 0 435 837

## Description

La présente invention se rapporte aux dispositifs qui permettent de comprimer de manière contrôlée le sein d'une patiente pour en faire une radio avec un mammographe.

Pour faire une radio des seins on utilise un appareil de radiographie spécial, appelé mammographe, qui permet grâce à un tube générateur de rayons X particulier d'effectuer les radios selon des angles permettant de détecter plus facilement les éventuelles anomalies. En outre le mammographe comprend des moyens de contention et de compression du sein qui permettent de le placer dans la position la meilleure correspondant à ces angles. Ces moyens comprennent une partie mobile, appelée "pelote", qui vient comprimer le sein sur le porte-plaque qui contient la plaque photographique sensible aux rayons X.

Pour ne pas traumatiser la patiente, il convient d'exercer avec la pelote une pression juste suffisante pour l'examen, mais qui ne provoque ni douleur ni traumatisme. Les systèmes utilisés jusqu'à présent comportent des vérins pneumatiques dont on mesure la pression de fonctionnement pour en déduire la force qui s'exerce sur le sein. Un tel système donne des indications peu précises, mal reproductibles et il est en outre assez brutal. Le confort de la patiente pendant l'examen n'est donc pas bon.

La demande de brevet européen EP-A-0 144 670 divulgue un dispositif permettant de mesurer la force de compression, comprenant un chariot mobile, une pelote mobile en rotation par rapport au chariot mobile, et des moyens de mesure de la force de compression à partir de la rotation de la pelote et du chariot.

L'invention propose un dispositif de compression pour mammographe, comprenant un chariot mobile entraînant une pelote de compression, des moyens pour entraîner en translation le chariot mobile, une partie fixe par rapport au chariot mobile, une partie mobile supportant la pelote de compression, des moyens apte à déduire la force de compression obtenue de la mesure du déplacement de ladite partie mobile par rapport à ladite portée fixe, principalement caractérisé en ce qu'il comprend un système de parallélogrammes articulés reliant la partie fixe à la partie mobile pour permettre entre celles-ci un mouvement relatif sensiblement équivalent à une translation parallèle à l'axe de translation du chariot mobile et à la direction de compression du sein.

D'autres particularités et avantages de l'invention apparaîtront clairement dans la description suivante, présentée à titre d'exemple non limitatif en regard des figures annexées qui représentent:
- la figure 1, une vue en perspective cavalière des organes mécaniques principaux d'un dispositif selon l'invention;
- la figure 2, une vue en coupe partielle des organes de la figure 1; et
- la figure 3, une variante de la coupe de la figure 2, comportant des organes de mesure.

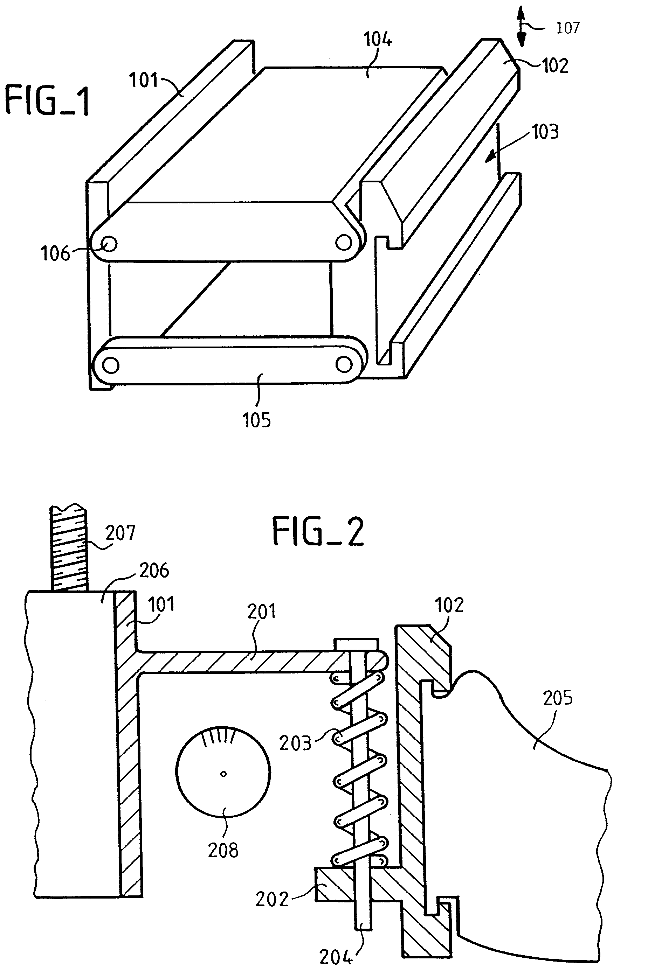

Le dispositif selon l'invention représenté en figure 1 comporte une partie 101 entraînée par un chariot fixé sur le corps du mammographe. Cette partie 101 sera appelée partie fixe pour les besoins de la description . Une partie 102, elle-même appelée partie mobile pour les besoins de la description, se déplace par rapport à la partie 101 dans un mouvement de translation 106 parallèle à l'axe de translation du chariot mobile et à la direction de compression du sein, avec une amplitude relativement faible. Cette partie 101 comporte vers l'extérieur une saignée 103 en queue d'aronde qui permet de fixer la pelote de compression destinée à comprimer le sein de la patiente. La liaison entre les parties 101 et 102 est effectuée par une chapeau 104 et deux biellettes dont on ne voit que la biellette 105 sur la figure.

Le chapeau 104 et les deux biellettes sont articulés respectivement sur la partie fixe 101 et sur la partie mobile 102 à l'aide d'axes 106 de manière à permettre le débattement de la partie mobile 102 par rapport à la partie fixe 101 selon la direction 107. Le chapeau 104 forme avec chacune des biellettes un parallélogramme déformable qui permet ce mouvement. En fait ce chapeau 104 joue le rôle de deux biellettes dont chacune forme avec l'une des biellettes simples l'un des parallélogrammes. On pourrait sans sortir du cadre de l'invention utiliser quatre biellettes, deux de chaque côté, mais il faudrait alors pour maintenir la rigidité en torsion de l'ensemble prévoir des biellettes plus épaisses et limiter strictement le jeu au niveau des articulations 106. La présence du chapeau permet donc d'alléger l'ensemble et de tolérer un jeu plus important au niveau de ces articulations, ce qui diminue les frottements et donc les efforts non compensables.

La partie fixe 101 comprend, comme représenté sur la coupe partielle de la figure 2, une ailette 201 qui s'étend vers la partie mobile 102 et qui se termine à une faible distance de celle-ci au-dessus d'un bossage 202 qui ressort de cette pièce 102 en s'étendant vers la pièce 101 sur une courte distance. Un ressort 203 est placé entre cette ailette et ce bossage en étant fixé respectivement à chacun de ces deux organes. Une tige 204 traverse ce ressort en étant fixée à l'un des deux organes, l'ailette 201 sur la figure. Elle coulisse dans un trou ménagé dans l'autre partie, le bossage 202 sur la figure. Cette tige sert de guidage au ressort 203 et évite que dans son mouvement de compression il ne fléchisse latéralement ce qui perturberait la liaison entre les deux pièces 101 et 102. Ce ressort 203 est fixé sur l'ailette 201 et sur le bossage 202 de manière à ce qu'il puisse travailler aussi bien en compression qu'en extension.

Dans sa position neutre, correspondant à un effort nul sur la pelote de compression 205 qui s'étend à l'extérieur de la pièce 102 en étant fixée dans la rainure 103, ce ressort maintient les pièces 101 et 102 l'une par rapport à l'autre de telle manière que le chapeau 104 et les biellettes 105 soient sensiblement perpendiculaires à ces deux pièces, les parallélogrammes d'articulation étant donc à ce moment très sensiblement rectangles. De cette manière le déplacement de la pièce 102, et donc de la pelote 205 qu'elle entraîne, par rapport à la pièce 101, et donc au bâti qui la supporte, correspond bien sensiblement à une translation parallèle au déplacement du chariot 206 sur lequel est fixée la pièce "fixe" 101, sans mouvement de rotation et pratiquement sans mouvement d'approche ou de recul entre les deux pièces.

Ainsi le système de mesure de la force de compression est bien indépendant du point d'application de la force, c'est à dire de la manière dont le sein de la patiente est pincé entre la pelote 205 et le porte-plaque du mammographe.

La partie "fixe" 101 est donc fixée sur le bâti du mammographe par l'intermédiaire du chariot mobile 206. Elle n'est donc en fait fixe que par rapport à ce dernier.

Celui-ci coulisse par exemple sur des glissières et est entraîné par un système symbolisé sur la figure par une vis 207 qui vient se visser dans le corps du chariot 206, en étant entraîné, par exemple, par un moteur pas à pas. Une variante de réalisation consisterait à utiliser une courroie fixée au chariot et elle-même entraînée par un moteur pas à pas. Le mouvement du chariot 206 est en général vertical, le sein étant maintenu au-dessus par la pelote et en-dessous par le porte plaque. Le moteur pas à pas permet d'assurer un asservissent en position parfait supprimant les risques de "bougés" pendant l'exposition. Ceci est un risque lorsque la pelote est contrôlée en pression et que le sein flue légèrement. Le moteur pas à pas permet aussi de contrôler en courant le couple maximal de maintien en position. La commande d'un moteur pas à pas offre beaucoup de sécurité en cas de premier défaut (aucun risque de compression incontrôlée) tout en conservant la reversibilité du mouvement.

Ce moteur pas à pas est actionné par un dispositif électronique contrôlé par un logiciel. Le logiciel contrôle le mouvement en fonction des commandes de l'opérateur en assurant de ne pas dépasser une force maximum présélectionnée. Le moteur reçoit des signaux de commande. Ces signaux de commandes sont obtenus dans l'exemple de réalisation décrit à partir d'une roue codeuse 208 fixée à la partie fixe 101. Cette roue codeuse est entraînée par un bouton moleté actionné par l'opérateur, lequel contrôle par ailleurs la force de compression exercée par la pelote 205.

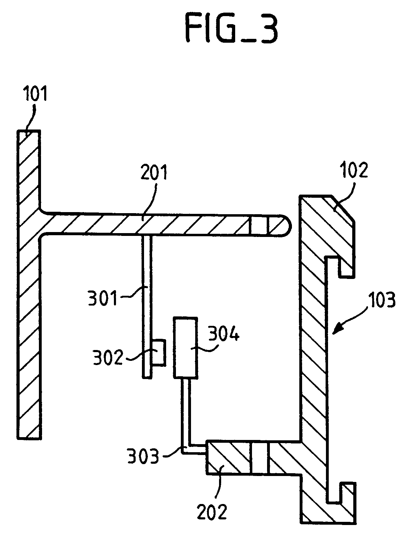

Le système de mesure de cette force comprend, figure 3, un capteur à effet Hall 302 fixé à la partie mobile 101 par l'intermédiaire de moyens de fixation 301. Devant ce capteur à effet Hall, dans sa zone de mesure, se trouve un aimant 304 fixé à la partie mobile 102 par l'intermédiaire de moyens de fixation 303. Ces organes sont situés, dans l'exemple décrit, dans l'espace intérieur compris entre les pièces fixe et mobile, et les moyens de fixation sont reliés d'une part à l'ailette 201 et d'autre part au bossage 202. Cet emplacement permet tout à la fois de protéger les organes et d'assurer un débattement correct sans interférence avec les autres parties du dispositif.

Dans son mouvement relatif par rapport à la pièce fixe, la pièce mobile 102 entraîne l'aimant 304 devant le capteur à effet Hall 302, ce qui, de manière connue, modifie la tension de sortie de ce capteur à effet Hall. On sait en effet qu'un tel capteur permet une mesure à la fois précise et linéaire de l'emplacement de l'aimant 304 par rapport à lui-même, sur une course de quelques millimètres. On peut ainsi mesurer le déplacement de la partie mobile par rapport à la partie fixe de manière précise. La raideur du ressort 203 est bien entendue calculée de manière à obtenir la pression maximale à exercer dans l'intervalle de déplacement autorisé par la plage de mesure du capteur.

Ainsi l'opérateur, après avoir placé la patiente en position sur le mammographe, rapproche tout d'abord dans un mouvement rapide la pelote du sein à examiner. Pour cela il peut éventuellement utiliser des moyens de commande plus rapides que la roue codeuse 208, par exemple un bouton de marche continue. Lorsque la pelote est suffisamment rapprochée, il fait alors déplacer l'ensemble de compression en manipulant la roue codeuse 208 tout en examinant les indications du système de mesure de la force visualisée sur un afficheur. Lorsque ces indications montrent que la pression suffisante est obtenue, il cesse d'actionner la roue codeuse et il peut alors passer à la phase d'examen radiographique proprement dite.

Le système de compression ainsi décrit permet donc de mesurer la force de compression indépendamment du point d'application de celle-ci de manière fiable et précise. Cette force d'application est réglable facilement en provoquant un minimum de gêne pour la patiente. Ce dispositif est facile à construire et son coût est faible.

A titre de variante, on pourrait relier les moyens de mesure aux moyens d'entraînement du chariot supportant le dispositif, à l'aide d'un système électronique de réglage comportant des moyens pour afficher par avance la force de compression souhaitée et des moyens pour actionner le système d'avance du chariot en fonction de cette mesure, de manière à obtenir automatiquement la valeur voulue pour cette force de compression.

## Revendications

1. Dispositif de compression pour mammographe comprenant un chariot mobile (206) entraînant une pelote de compression (205), des moyens (207) pour entraîner en translation le chariot mobile, une partie fixe (101) par rapport au chariot mobile, une partie mobile (102) supportant la pelote de compression, et des moyens (302,304) aptes à déduire la force de compression obtenue de la mesure du déplacement relatif de ladite partie mobile par rapport à ladite partie fixe, caractérisé en ce qu'il comprend un système de parallélogrammes articulés (104, 105) reliant la partie fixe à la partie mobile pour permettre entre celles-ci un mouvement relatif (106) sensiblement équivalent à une translation parallèle à l'axe de translation du chariot mobile et à la direction de compression du sein.

2. Dispositif selon la revendication 1, caractérisé en ce que le système de parallélogrammes articulés est formé d'un chapeau (104) articulé d'un côté en deux points sur la partie fixe et de l'autre en deux points sur la partie mobile, et de deux biellettes (105) articulées chacune d'un côté en un point sur la partie fixe et de l'autre en un point sur la partie mobile; le chapeau formant avec chacune des biellettes un parallélogramme articulé.

3. Dispositif selon l'une quelconque des revendications 1 et 2, caractérisé en ce que la partie fixe et la partie mobile sont reliées par un ressort (203) qui dans sa position d'équilibre maintient ces parties entre elles de manière à ce que le système de parallélogrammes articulés (104,105) soit sensiblement rectangle ; la raideur de ce ressort déterminant la force de compression souhaitée en fonction de l'amplitude maximale admise pour le mouvement de la pièce mobile par rapport à la pièce fixe.

4. Dispositif selon l'une quelconque des revendications 1 à 3, caractérisé en ce qu'il comprend une jauge à effet Hall (302) fixée à l'une des deux pièces fixe ou mobile, et un aimant (304) fixé à l'autre de ces deux pièces de manière à se trouver dans la zone de mesure de la jauge ; cette jauge et cet aimant permettant de mesurer le déplacement relatif de la pièce mobile par rapport à la pièce fixe et d'obtenir, compte-tenu de la raideur du ressort, la valeur de la force de compression exercée.

5. Dispositif selon l'une quelconque des revendications 1 à 4, caractérisé en ce que les moyens pour entraîner en translation le chariot mobile comprennent une roue codeuse (208) permettant de contrôler le fonctionnement d' un moteur pas à pas qui entraîne (207) le chariot.

6. Dispositif selon la revendication 5, caractérisé en ce que le chariot est entraîné par une courroie (207).

7. Dispositif selon la revendication 5, caractérisé en ce que le chariot est entraîné par une vis sans fin (207).

8. Dispositif selon l'une quelconque des revendications 4 à 7, caractérisé en ce que les moyens de mesure (302,304) de la force de compression sont reliés aux moyens d'entraînement (207) du chariot mobile par un dispositif électronique contrôlé par un logiciel pour limiter automatiquement la force de compression à une valeur présélectionnée.

## Claims

1. Compression device for mammography equipment comprising a moving carriage (206) driving a compression pad (205), means (207) for driving the moving carriage in translational movement, a part (101) which is fixed with respect to the moving carriage, a moving part (102) supporting the compression pad, and means (302, 304) able to derive the compression force obtained from the measurement of the relative movement of the said moving part with respect to the said fixed part, characterised in that it comprises a system of articulated parallelograms (104, 105) connecting the fixed part to the moving part in order to permit a relative movement (106) of the said parts which is substantially equivalent to a translational movement parallel to the translation axis of the moving carriage and to the direction of compression of the breast.

2. Device according to Claim 1, characterised in that the system of articulated parallelograms is formed by a hood (104) articulated on one side at two points on the fixed part and on the other side at two points on the moving part, and two bars (105) each articulated on one side at one point on the fixed part and on the other side at one point on the moving part; the hood forming with each of the bars an articulated parallelogram.

3. Device according to either one of Claims 1 and 2, characterised in that the fixed part and the moving part are connected by a spring (203) which, when in its equilibrium position, holds these parts in position with respect to each other so that the system of articulated parallelograms (104, 105) is substantially rectangular; the rigidity of this spring determining the desired compression force in accordance with the maximum permitted amplitude for the movement of the moving part with respect to the fixed part.

4. Device according to any one of Claims 1 to 3, characterised in that it comprises a Hall effect gauge (302) fixed to one of the two fixed or moving parts, and a magnet (304) fixed to the other of these two parts so as to be located in the measuring area of the gauge, this gauge and this magnet making it possible to measure the relative movement of the moving part with respect to the fixed part and to obtain, taking into account the rigidity of the spring, the value of the compression force exerted.

5. Device according to any one of Claims 1 to 4, characterised in that the means for driving the moving carriage in translational movement comprise a knurled wheel (208) for controlling the functioning of a stepping motor which drives (207) the carriage.

6. Device according to Claim 5, characterised in that the carriage is driven by a belt (207).

7. Device according to Claim 5, characterised in that the carriage is driven by a worm screw (207).

8. Device according to any one of Claims 4 to 7, characterised in that the means (302, 304) for measuring the compression force are connected to the means (207) for driving the moving carriage by an electronic device controlled by software to limit the compression force automatically to a preselected value.

## Patentansprüche

1. Kompressionseinrichtung für einen Mammograph umfassend einen bewegbaren Schlitten (206), der ein Kompressionskissen (205) mitnimmt, Mittel (207) für die Translationsbewegung des bewegbaren Schlittens, einen festen Teil (101) in bezug auf den bewegbaren Schlitten, ein bewegbares Teil (102), der das Kompressionskissen trägt und Mittel (302, 304), die geeignet sind die Kompressionskraft abzuleiten, die erhalten wird durch Messung der Relativbewegung zwischen dem bewegbaren Teil in bezug auf den festen Teil, dadurch gekennzeichnet, daß sie ein mit Gelenken versehenes Parallelogrammsystem (104, 105) umfaßt, welches das feste Teil mit dem bewegbaren Teil verbindet, um zwischen ihnen eine Relativbewegung (107) zuzulassen, die im wesentlichen einer Translationsbewegung parallel zur Translationsbewegung des bewegbaren Schlittens und zur Kompressionsrichtung der Brust ist.

2. Einrichtung nach Anspruch 1, dadurch gekennzeichnet, daß das mit Gelenken versehene Parallelogrammsystem gebildet ist aus einer Platte (104), die einerseits an zwei Punkten an dem festen Teil und andererseits an zwei Punkten an dem bewegbaren Teil angelenkt ist und aus zwei Lenkern (105), die jeweils einerseits an einem Punkt an dem festen Teil und andererseits an einem Punkt am bewegbaren Teil angelenkt sind; wobei die Platte zusammen mit den Lenkern ein Gelenkparallelogramm bilden.

3. Einrichtung nach einem der Ansprüche 1 und 2, dadurch gekennzeichnet, daß das feste Teil und das bewegbare Teil durch eine Feder (203) miteinander verbunden sind, die in der Gleichgewichtsstellung diese Teile so zueinander hält, daß das mit Gelenken versehene Parallelogrammsystem (104, 105) im wesentlichen rechtwinklig ist; wobei die Steifigkeit der Feder die gewünschte Kompressionskraft in Abhängigkeit von der maximal zugelassenen Bewegungsamplitude des bewegten Teiles in bezug auf das feste Teil bestimmt.

4. Einrichtung nach einem der Ansprüche 1 bis 3, dadurch gekennzeichnet, daß sie eine Halleffekt-Meßeinrichtung (302), die an einem der beiden Teile, fest oder beweglich, befestigt ist und einen Magneten (304) umfaßt, der an dem anderen der beiden Teile derart befestigt ist, daß er sich in der Meßzone der Meßeinrichtung befindet; wobei diese Meßeinrichtung und der Magnet die Relativverschiebung des beweglichen Teils gegenüber dem festen Teil zu messen und im Hinblick auf die Steifigkeit der Feder den Wert der ausgeübten Kompressionskraft zu erhalten gestattet.

5. Einrichtung nach einem der Ansprüche 1 bis 4, dadurch gekennzeichnet, daß die Mittel für die Translationsbewegung des bewegbaren Schlittens ein Kodierrad (208) umfaßt, welches die Steuerung eines Schrittmotors gestattet, der den Schlitten (207) antreibt.

6. Vorrichtung nach Anspruch 5, dadurch gekennzeichnet, daß der Schlitten durch einen Treibriemen (207) antreibbar ist.

7. Vorrichtung nach Anspruch 5, dadurch gekennzeichnet, daß der Schlitten durch eine Schraube ohne Ende (207) antreibbar ist.

8. Einrichtung nach einem der Ansprüche 4 bis 7, dadurch gekennzeichnet, daß die Mittel (302, 304) zur Messung der Kompressionskraft mit den Antriebsmitteln (207) des bewegbaren Schlittens durch eine elektronische Einrichtung verbunden sind, die durch eine Software gesteuert ist, um automatisch die Kompressionskraft auf einen vorbestimmten Wert zu begrenzen.
